# EUROPEAN PATENT SPECIFICATION

(11) **EP 3 196 644 B1**
(45) Date of publication and mention of the grant of the patent: **27.11.2019**
(21) Application number: 16152392.3
(22) Date of filing: 22.01.2016
(51) Int. Cl.: G01N 33/04

(54) **METHOD FOR DIAGNOSING MASTITIS IN A LACTATING ANIMAL**
VERFAHREN ZUR DIAGNOSE VON MASTITIS BEI LAKTIERENDEN TIEREN
PROCÉDÉ PERMETTANT DE DIAGNOSTIQUER UNE MASTITE CHEZ UN ANIMAL EN LACTATION

(43) Date of publication of application: 26.07.2017
(73) Proprietor: Mastatix, Ltd., Ithaca, NY 14852 (US)
(72) Inventor: QUIMBY, Fred William, New Durham NH 03855 (US); VON RHEINBABEN, Rolf, 82327 Tutzing (DE); TOKMAN, Michael Gregory, Ithaca NY 14850 (US)
(74) Representative: Frick, Robert

(56) References cited:
- EP-A1- 1 540 336
- WO-A1-2005/034970
- WO-A1-2010/142301
- WO-A1-2012/052046
- US-A- 5 416 417
- US-A1- 2013 197 811

## Description

Infectious bovine mastitis is a major health problem of dairy cattle which results in decreased milk production and decreased milk quality.

Identification of bovine mastitis has historically been based on counting of all cells present in milk (leukocytes and epithelial cells), also known as somatic cell counts (SCC). However, diagnosis based on SCC is inaccurate, as it does not take into account the contribution of different cell types.

Different methods have been developed in the past to improve diagnosis of mastitis. Among these methods are those described in US 6,979,550 B1 and in US 2013/0197811 A1, which use the differential leukocyte count in a single sample of milk to identify presence and stage of mastitis.

In Y.deHaas, RF Veerkamp, HW Barkema, YT Grohn, and YH Schukken: "Associations between pathogen-specific cases of clinical mastitis and somatic cell count patterns", Journal of Dairy Science 87: 95-105, 2004, a retrospective analysis was conducted to determine if changes in the pattern of somatic cells count, calculated from composite milk collected daily from dairy cows, could predict clinical infection in at least one quarter and predict the causative bacterial agent. Five different SCC patterns were compared over a 5-day period around the time of infection. Correlations were sought between SCC pattern and one of 5 types of bacteria cultured at the infection site. They found that a single SCC pattern was significantly associated with infection by E.coli and another pattern for S.aureus but the positive predictive value and the sensitivity of the tests (patterns) were low in both cases. No significant associations were seen between SCC pattern and any of the three remaining bacteria.

The purpose of the present invention is to provide an improved method which allows for a more accurate and timely diagnosis of mastitis.

Against this background, the invention pertains to a method for diagnosing mastitis in a lactating and preferably bovine animal, the method comprising the steps disclosed in claim 1.

As understood by claim 1, the total leukocyte count may be the 'true' total leukocyte count or the somatic cell count.

One key aspect of the invention is that it describes a longitudinal method, where several measurements are carried out on the same animal over time (e.g., daily) and changes in leukocyte counts and preferably differential leukocyte counts are used to establish the diagnosis. To the contrary, the methods of US 6,979,550 B1 or US 2013/0197811 A1 use data taken at one fixed point in time.

Another aspect of the invention is that it uses a differential leukocyte count including the total leukocyte count (TL count or TLC) and the counts of the most common leukocytes, which are the lymphocytes (L), the macrophages (M) and the neutrophils (N). Lymphocytes are cells of adaptive immunity, macrophages are cells which remove dead and dying cells and debris including dead cells which compose the mammary gland and neutrophils are cells which identify and kill bacteria. The total leukocyte count is the sum of lymphocyte, macrophages and the neutrophils count per unit volume of milk. Alternatively, the method uses SCC as total leukocyte count and the explanations hereinafter are also applicable when using the SCC instead of the TLC. As in infected animals most of the somatic cells in milk are leukocytes, these values are similar.

According to the invention, evaluating the recorded data includes determining mean values and confidence intervals for the total and each of the differential leukocyte counts.

The mean values and confidence intervals may together be referred to as baseline. As a mean value, the arithmetic mean of the predetermined number of samples may be used. As confidence interval, the 95 % confidence interval may be used.

Beyond the scope of the invention, this step is only carried out in case all counts (TL, L, M and N) remain stable and the TL count remains below the upper boundary value over the predetermined minimum number of consecutive samples. This number may be between 5 and 15 and preferably lies between 7 and 10. A count may in one embodiment be considered stable if it does not change by more than 50% between consecutive samples. The upper boundary value for the TLC may be at 600. Generally, the cell count numbers are given herein as multiples of 10³ cells per ml of milk. A TLC of 600 hence means that there are 600,000 leukocytes per ml of milk. If reference is made to an N, L or M "percentage", this refers to the percentage of the respective leukocyte based on the TLC.

In one embodiment, the mean value and confidence interval may be updated by using the count(s) of new samples taken after the predetermined minimum number has already been reached. Optionally, the mean value and the confidence interval may also be updated by dismissing old counts, provided that the predetermined minimum number of samples remains under consideration. This way, only the most current prior observations will be used to make future predictions.

In one embodiment, evaluating the recorded data further includes evaluating whether the count(s) of a new sample remain within the respective confidence intervals and, if applicable, identifying a deviation.

In this embodiment the counts of new samples, i.e., samples taken after the baseline has been established, are compared to the existing baseline. An animal (quarter) is considered healthy if the counts do not deviate from the baseline and the baseline mean is below TLC 400. This will normally be the case for most animals throughout the entire lactation period.

In one embodiment, evaluating the recorded data further includes evaluating the deviation to identify acute infection, chronic infection or recovery. Analyzing the deviation may include evaluating the direction and magnitude of the deviation.

In one embodiment, the method further includes identifying and outputting recommendations on whether to dump the milk or treat the animal on the basis of the result of the evaluation of the deviation.

As such, according to the invention, after each milk collection, and while the cow is still being milked, the present values for the absolute number of the three leukocyte populations may be computed and compared to the baseline and confidence intervals. Values for any leukocyte population which falls above or below the 95% confidence interval may then prompt a decision tree analysis to make a decision on the disposition for the milk and the cow/quarter. If the cell populations in the current milk being collected do not fall above or below the 95% confidence interval, no decision tree analysis may be called up and the new values for each leukocyte population are added to the previous ones, the earliest stored value is dropped, and the mean and 95% confidence interval are recalculated.

According to the invention, evaluating the recorded data further includes assigning the samples to threshold groups based on the mean value of the total leukocyte count or somatic cell count. The method may, for example, use five threshold groups based on the TLC. These groups may in one embodiment be: "normal" (TLC≤150), "subclinical" (151 <TLC≤400), "chronic 1" (400<TLC≤600), "chronic 2" (600<TLC≤800) and "acute" (TLC>800).

According to the invention, evaluating the recorded data further includes evaluating whether the total leukocyte count (or somatic cell count) of a new sample enters a higher threshold group and, if applicable, which threshold group has been entered.

Therefore it is checked whether new samples would have to be assigned to higher threshold groups than suggested by the baseline. An animal (quarter) is considered healthy if this is not the case, which is expected for most animals throughout the entire lactation period.

In one embodiment, evaluating the recorded data further includes evaluating the change of threshold groups to identify acute infection, chronic infection or recovery. In one embodiment, the method further includes identifying and outputting recommendations on whether to dump the milk or treat the animal on the basis of the result of evaluating the change of threshold groups.

The method may hence rely on a decision tree analysis comprising thresholds, which may be used for the preliminary categorization of the health status of the quarter and whether or not the milk collected should be added to or removed from the human milk stream.

According to the invention, evaluating the recorded data further includes evaluating whether a threshold group has been exceeded and, if so, evaluating the recorded data to obtain information on the microorganism responsible for mastitis. Based on the historic information regarding peak TLC, length of the plateau before the TLC decreases, and the change in percentages of N, M and L during a number of previous samplings, a prediction of a specific microorganism is made.

In one embodiment, the period for milking is once or twice daily. Milking to obtain subsequent samples for the longitudinal sequence of data hence, on average and under normal circumstances, occurs in 24 hour intervals. Individual intervals may differ from this average, e.g., lie anywhere between 20 and 28 hours. In case mastitis is detected, the routine may be switched to 12 hour intervals instead to allow reaching a diagnosis faster. If identification of the microorganism responsible for mastitis is intended, 12 hour intervals may be preferred. If presence and/or stage of the mastitis shall be diagnosed, 24 hour intervals may be preferable from an economic standpoint.

In one embodiment, the TLC/SCC count and preferably differential leukocyte count is determined by flow cytometry. This method is capable of real-time determination of the total leukocyte count, the lymphocyte count, the macrophage count and the neutrophil count in milk from a small sample of the milk. In this method, cells in milk are stained with a fluorescent dye and subjected to excitation by a laser where emissions from the fluorescent dye are collected and the cells represented by the fluorescent emissions are further analysed by side angle scatter and forward angle scatter in order to characterize each fluorescent emitting cell into one of three leukocyte populations. While this is not the only possible method for such analysis it appears to be one which will allow real-time processing in the time the cow is being milked (lasting about 10 minutes).

In one embodiment, individual series of samples are obtained for different quarters. This means that all steps of the method are carried out on the basis of samples obtained individually for different quarters, such that the diagnosis is made not only for the animal but individually for each of these mammary gland sections/for each teat.

In a different embodiment, the samples constitute composite samples comprising milk from each of the four quarters of a cow.

Milking may be carried out using automated milking machines. An automated milking machine is a system in which an animal, usually upon it's own will, enters an automated milker which disinfects the udder, attaches suction devices to each quarter and applies vacuum allowing for collection of milk. Milk may be collected separately for each quarter and stored separately for each quarter and animal.

The invention further pertains to a data storage medium (e.g., a data DVD) comprising a computer-readable program configured to carry out the method of the invention and a computer configured to carry out the method of the invention.

Further details and advantages of the invention become apparent from the following example. The figures show:
- Figure 1:: a flow chart providing an overview on the DIM calculator subroutine of a method of the invention;
- Figure 2:: a flow chart describing a first subroutine ("SR1"), which is responsible for establishing a baseline;
- Figure 3:: a flow chart describing a transitional subroutine ("SR1a");
- Figure 4:: a flow chart describing a second subroutine ("SR2"), which is triggered to follow the leukocyte profile during recovery and self cure;
- Figure 5:: a flow chart describing a third subroutine ("SR3"), which is triggered to follow the leukocyte profile when an infection is suspected;
- Figure 6:: a flow chart describing a variant of the third subroutine ("SR3a"), which is triggered in case the threshold group has not changed;
- Figure 7:: a flow chart describing a fourth subroutine ("SR4"), which is triggered to establish a species specific diagnosis; and
- Figure 8:: a flow chart describing a fifth subroutine ("SR5"), which is triggered if a deviation from a previously established baseline occurs.

As a quarter is milked in an automated milking machine, the cows ID is read using radiofrequency identification. Cows are commonly identified using a radiofrequency chip implanted subcutaneously (typically in one ear). Stored information on the cow is pulled up. Among these data are the cows DIM ("days in milk" number) and the previous TL, L, M and N counts. The DIM number designates the number of the actual day starting form the beginning of the lactation cycle.

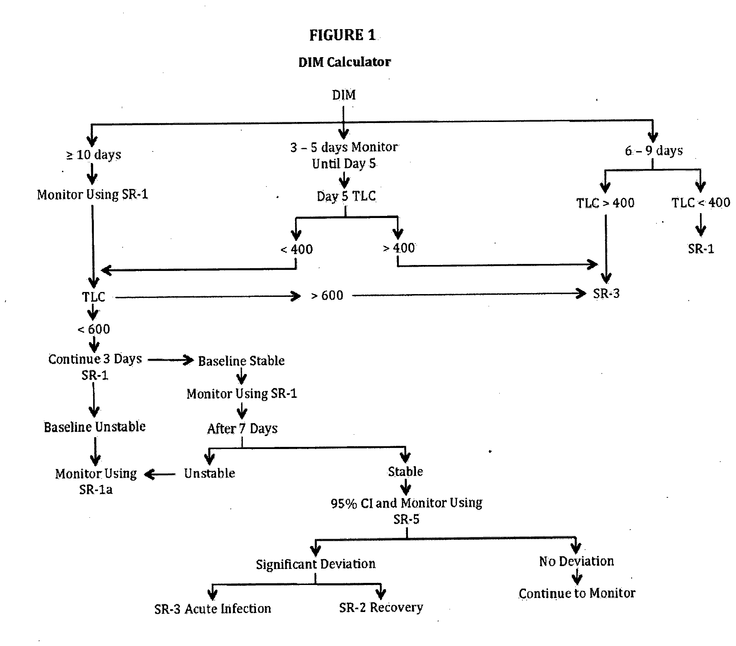

As apparent from Figure 1, a routine called the DIM calculator is then initiated, which decides the further steps to be taken on the basis of the DIM. If the DIM is less than 3 the milk is not analyzed. The reason is that following calving there is a physiological response in the cow which causes neutrophils from the blood to enter the milk in all quarters in very high numbers (N>1,000). This is a normal protective response and the N count falls rapidly in the subsequent days until it plateaus out at the background count (usually at around DIM 5). Quarters with sustained high N counts (greater than 400) after DIM 5 should be considered infected.

The first analysis is recorded at DIM 3. After collecting and analyzing milk from DIM 3 to 5 the calculator determines whether or not the quarter is likely to be infected. For those quarters likely to be infected at DIM 5 the calculator initiates the subroutine 3 ("SR3", see later) to follow the leukocyte patterns and terminates. The reason for this is that no normal cow after DIM 5 has a TLC in excess of 400 cells/ml without an infection, therefore this quarter will be monitored as if it were infected. Early lactation is a critical period for mastitis on a dairy due to the trauma of delivery, metabolic changes occurring at the onset of lactation and the conversion of the mammary gland from quiescent to productive. Over 30% of all new infections of the quarter take place in the first few DIM.

Any animal introduced into the herd after DIM 3 has its quarter milk information introduced directly into subroutine 1 ("SR1", see later) establishing the baseline parameters for milk leukocytes. After DIM5 if the TLC is below 400,000 cells/ml the quarter is monitored to establish the baseline for the quarter (SR1).Any cow entering the milking herd which is greater than DIM5 and has a TLC < 600,000 cells/ml is also monitored for baseline.

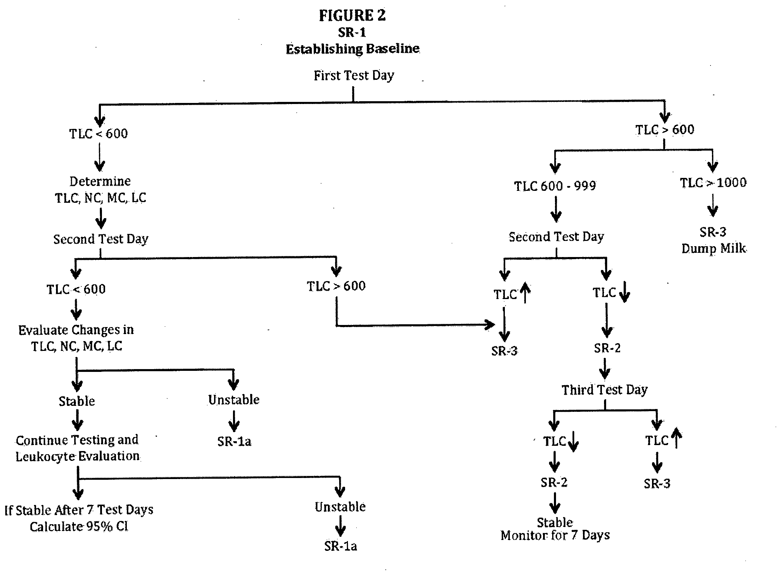

Figure 2 illustrates a decision tree describing the first subroutine ("SR1"), which is used according to the invention to establish an initial or a new baseline.

The first sample subject to SR1 is analyzed to determine TLC. If TLC is below 600, SR1 determines the individual L, M and N counts and the relative percentages of each leukocyte type and then enters a baseline establishing step, wherein it remains active until a further milk sample is analyzed and compares TLC and the individual L, M and N counts or percentages of the further sample.

If TLC of the further sample remains below 600 if all four counts remain stable in the sense that no change of more than 50% over the first sample is observed, SR1 repeats the baseline establishing step. If the baseline establishing step can be repeated until seven subsequent sets of all four counts are obtained, SR1 establishes a baseline, i.e., the means and 95% confidence intervals for all four counts. Once the baseline is established the quarter MDLC is monitored daily until a significant deviation from baseline in the sense that either of the four counts falls outside the confidence intervals is detected. As long as no such deviation is detected, the animal is regarded healthy. If such deviation is detected, the routine initiates SR5.

Should the TLC in the first sample (or any sample before a baseline is established) lie between 600 and 1000, SR1 continues to evaluate the TLC for the next sample. Should the TLC in the next sample have increased, SR1 initiates SR3, produces an output to discard the milk and terminates. Should the TLC in the next sample have decreased, SR1 initiates SR2 and terminates.

Should the TLC in the first sample (or any sample before a baseline is established) lie above 1000, SR1 initiates SR3, produces an output to discard the milk and terminates.

Should the TLC stay below 600 but any of the four counts become unstable before a baseline is established, SR1 initiates SR1a and terminates.

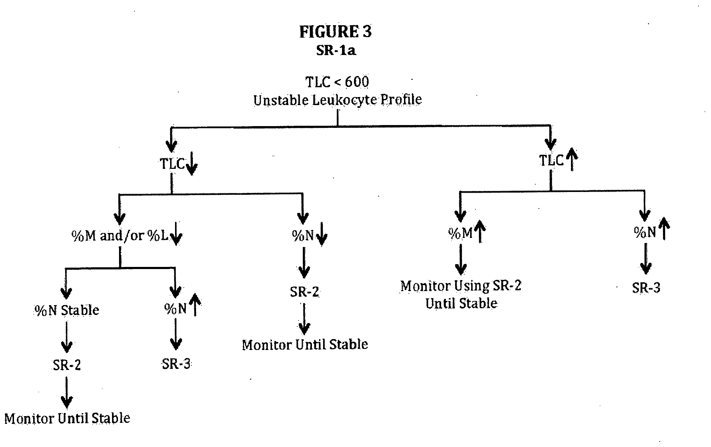

Figure 3 illustrates a decision tree describing Subroutine 1a ("SR1a"), which is a transitional subroutine which triggers SR2 or SR3 starting from SR1. SR1a first evaluates whether the TLC of the unstable sample has increased as compared to the previous sample. If so, SR1a initiates either SR3 (if the N percentage has increased) or SR2 (if the M percentage has increased) and terminates. If not, SR1a initiates either SR2 (if the N percentage has remained stable or decreased) or SR3 (if the N percentage has increased and only the L or M percentage has decreased) and terminates.

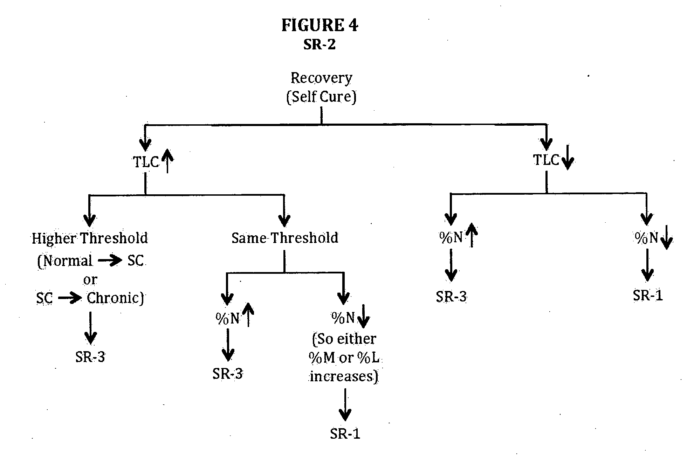

Figure 4 illustrates a decision tree describing subroutine 2 ("SR2"), which is initiated by the method of the invention to follow the leukocyte profile during recovery and self cure. It compares the TLC of the sample under observation to the TLC of the previous sample.

If the TLC has increased and changed into a higher threshold group (e.g., from "normal" to "subclinical" or from "subclinical" to "chronic 1"), SR2 initiates SR3 and terminates.

If the TLC has increased but not changed into a higher threshold group, SR2 evaluates whether the N percentage has increased. If yes, SR2 initiates SR3 and terminates. If no, SR2 restarts. If the N percentage falls and either the M percentage or L percentage increases,SR2 initiates SR1 and terminates.

If the TLC has decreased, SR2 evaluates whether the N percentage has increased. If yes, SR2 initiates SR3 and terminates. If no, SR2 initiates SR1 and terminates.

Subroutines 3 and 3a ("SR3" and "SR3a") are initiated to follow the leukocyte profile when an infection is suspected. Whether to apply SR3 or SR3a depends on whether the TLC value of the sample under observation has increased over the previous sample and changed into a higher threshold group. If yes, SR3 is initiated. If no, SR3a is initiated.

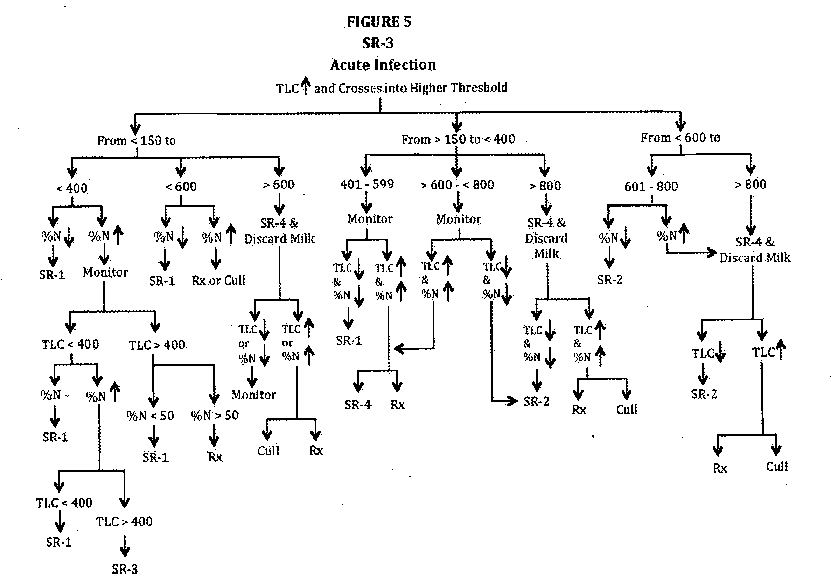

Figure 5 illustrates a decision tree describing subroutine 3 ("SR3"). This subroutine differentiates between eight possible scenarios.

In a first scenario the previous threshold group was normal (TLC<150) and the new threshold group is subclinical (150-400). In this scenario SR3 determines whether the N percentage has also increased. If not, SR3 initiates SR1 and terminates. If so, SR3 remains active until a further milk sample is analyzed, i.e., enters a monitoring routine. If the TLC of the further milk sample rises above the threshold group boundary (instantly: 400) and the N percentage is above 50, SR3 produces an output to treat the animal. If not, SR3 determines whether the N percentage has increased. If not, SR3 initiates SR1 and terminates. If so, SR3 remains active and the monitoring routine is repeated or treatment may be instituted.

In a second scenario the previous threshold group was normal (TLC<150) and the new threshold group is chronic 1 (400-600). In this scenario SR3 determines whether the N percentage has increased. If so, SR3 produces an output to treat the animal. If not, SR3 enters a monitoring routine as described for the first scenario.

In a third scenario the previous threshold group was normal (TLC<150) and the new threshold group is chronic 2 or acute (>600). In this scenario SR3 initiates SR4 and produces an output to discard the milk. Additionally, SR3 enters a monitoring routine. If the TLC or N percentage of the further milk sample still rises, SR3 produces an output to treat the animal. If not, SR3 initiates SR2 and terminates.

In a fourth scenario the previous threshold group was subclinical (150-400) and the new threshold group is chronic 1 (400-600). In this scenario SR3 enters a monitoring routine. If the TLC or N percentage of the further milk sample still rises, SR3 produces an output to treat the animal. If not, SR3 initiates SR1 and terminates.

In a fifth scenario the previous threshold group was subclinical (150-400) and the new threshold group is chronic 2 (600-800). Also in this scenario SR3 enters a monitoring routine. If the TLC or N percentage of the further milk sample still rises, SR3 produces an output to discard the milk and to treat the animal. If not, SR3 initiates SR2 and terminates.

In a sixth scenario the previous threshold group was subclinical (150-400) and the new threshold group is acute (>800). In this scenario SR3 initiates SR4 and produces an output to discard the milk. Additionally, SR3 enters a monitoring routine. If the TLC or N percentage of the further milk sample still rises, SR3 produces an output to treat the animal. If not, SR3 initiates SR2 and terminates.

In a seventh scenario the previous threshold group was chronic 1 (400-600) and the new threshold group is chronic 2 (600-800). In this scenario SR3 determines whether the N percentage has increased. If not, SR3 initiates SR2 and terminates. If so, SR3 produces an output to discard the milk. Additionally, SR3 enters a monitoring routine. If the TLC of the further milk sample still rises, SR3 produces an output to treat the animal. If not, SR3 initiates SR2 and terminates.

In an eighth scenario the previous threshold group was chronic 1 (400-600) and the new threshold group is acute (>800). In this scenario SR3 initiates SR4 and produces an output to discard the milk. Additionally, SR3 enters a monitoring routine. If the TLC of the further milk sample still rises, SR3 produces an output to treat the animal. If not, SR3 initiates SR2 and terminates.

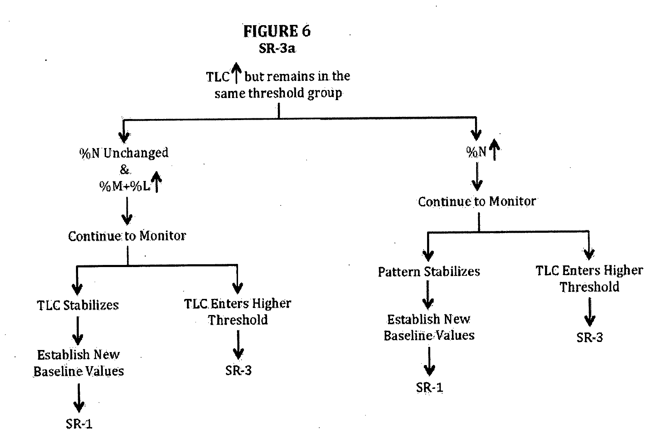

Figure 6 illustrates a decision tree describing subroutine 3a ("SR3a"), which first evaluates whether the N percentage has increased and then enters a monitoring routine. If the TLC of the further milk sample rises above the upper limit of the instant threshold group, SR3a initiates SR3 and terminates. If not, SR3 initiates SR1 and terminates.

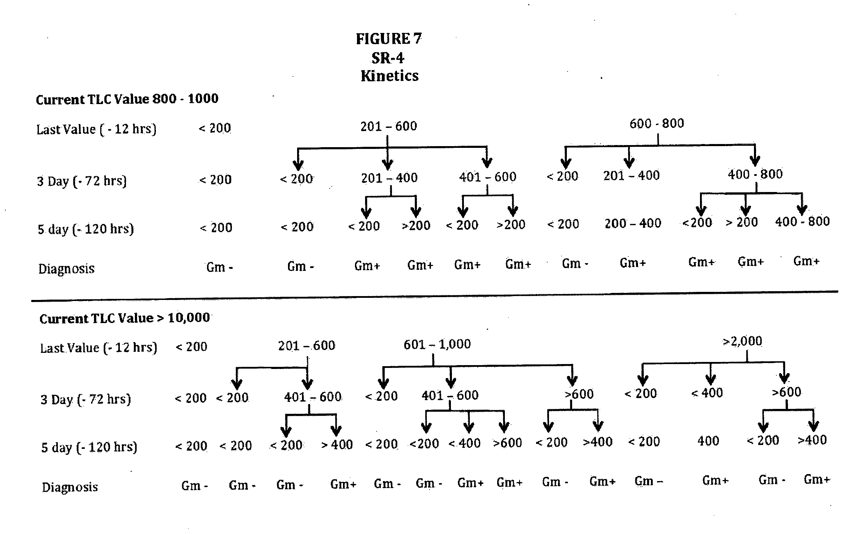

Figure 7 illustrates a decision tree describing subroutine 4 ("SR4"), which constitutes one key aspect of the present invention. It is initiated when TLC transcends more than one threshold group within 24 hours to establish a species specific diagnosis.

In the following, SR4 is explained on the basis of a 12 hour sampling frequency, assuming that the sampling frequency is increased after mastitis is detected.

This subroutine uses the previous value (12 hours ago), the value from three days ago and a value from five days ago to make a prediction, based on statistical information, whether mastitis is caused by gram positive (Gm+) or gram negative (Gm-) bacteria.

The prediction schemes for two different scenarios (800≤TLC<1,000 and TLC≥1,000) are illustrated in Figure 7.

For example, if the instant TLC is 900, the TLC of the previous sample was 700 and the TLCs from both three and five days ago were 100, the infection is due to gram negative bacteria. This information is provided to the user.

Furthermore, based on the historic information regarding peak TLC, length of the plateau before the TLC decreases, and the change in percentages of N, M and L during the previous three samplings, a prediction of a specific bacterial species may be made.

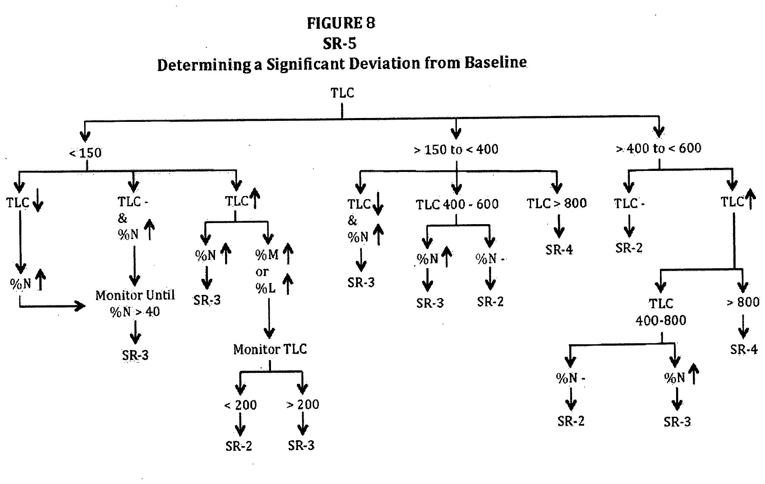

Figure 8 illustrates a decision tree describing subroutine 5 ("SR5"), which is initiated if a deviation from a previously established baseline occurs. This is the case when one of the four counts of a new sample falls outside the 95% confidence intervals of an existing baseline.

This subroutine differentiates between three possible scenarios.

In a first scenario, where the baseline mean TLC was 150 or smaller ("normal"), SR5 first determines whether the TLC has risen beyond, remained within or fallen below the confidence interval and subsequently determines whether the N percentage has risen beyond, remained within or fallen below the confidence interval. If both the TLC count and the N percentage have risen beyond the confidence interval, SR5 initiates SR3 and terminates. If TLC has risen beyond the confidence interval but the N percentage has not risen, SR5 enters a monitoring routine to check whether TLC becomes greater 200. If yes, SR5 terminates and initiates SR3. If no, SR5 terminates and initiates SR2. If TLC has remained within or fallen below the confidence interval but the N percentage has risen beyond the confidence interval, SR5 enters a monitoring routine to check whether the N percentage becomes greater 40. If yes, SR5 terminates and initiates SR3. If no, SR5 terminates and initiates SR2.

In a second scenario, where the baseline mean TLC was between 150 and 400 ("subclinical"), SR5 first determines whether the TLC of the new sample is below 800. If not, SR5 initiates SR4, produces a signal to discard the milk and treat the cow and terminates. If yes, SR5 next determines whether the N percentage has increased. If yes, SR5 initiates SR3 and terminates. If no, SR5 terminates and initiates SR2.

In a third scenario, where the baseline mean TLC was between 400 and 600 ("chronic 1"), SR5 first determines whether the TLC of the new sample has risen beyond the confidence interval. If so, SR5 next evaluates whether the TLC of the new sample has remained below 800. If not, SR5 initiates SR4, produces a signal to discard the milk and treat the cow and terminates. If yes, SR5 next determines whether the N percentage has increased. If yes, SR5 initiates SR3 and terminates. If no, SR5 terminates and initiates SR2.

To summarize the exemplary procedure starting from SR1, the daily counts are compared to the previous counts. If any leukocyte value increases or decreases by 50% in a 24 hour period it is considered unstable and monitoring continues unless the TLC exceeds 600 in which case the next 5 daily test TLC will either rise or fall calling for implementation of SR3 (acute infection) or SR2 (recovery)respectively. If however, the TLC never rises to 600 monitoring continues until the values become stable. After 7 test days in the stable condition the means and 95% confidence intervals for each value (L, N, M and TL count) is determined, this is referred to as the baseline for the quarter. Once the baseline is established the quarter counts are monitored daily until a significant deviation from baseline is detected (SR5).

If, while establishing baseline, the quarter TLC rises above 600 but less than 1,000 the next test will determine if SR2 (recovery) or SR3 (acute) is employed as a subroutine. Should the quarter rise to >1,000 or an N count greater than 600, it is immediately subjected to SR4 (leukocyte kinetics) monitoring.

This is because once the TLC exceeds 600 one of several phenomena occurs. First there may be an infection in another quarter which is causing an elevation in the neutrophil count of the monitored quarter. Second this quarter may be beginning an infection and must be watch carefully to document whether or not the infection remains subclinical, develops into a chronic disease, becomes worse, or goes into recovery. The object here is to prevent clinical mastitis which usually occurs when the TLC exceeds 2000. The program will be queried here to see if any other quarter has been diagnosed as mastitic in the previous 7 days, if mastitis is present, this quarter should recover after the infected quarter is treated and followed using SR2. If, however, there is no other indication for the elevated neutrophil count or the neutrophil count in the observed quarter does not decrease after treatment of the adjacent infected quarter, the quarter should be presumed infected and monitored using SR3.

The SR3 subroutine is used for unstable counts and high TLC in quarters without a baseline or when a quarter with a stable baseline shows a significant deviation from baseline. It is based on the concept that quarters may undergo transient infections in and out of thresholds considered subclinical (150-400) or they may progress into a chronic infection (400-600) before becoming acutely mastitic and clinical. SR3 follows the daily increases (or decreases) in leukocytes to determine if a higher category of infection is imminent. Changes in TLC and especially N count which cross over more than a single threshold in 24 hours have a high rate-of-change consistent with acute infection which must be treated. This high rate of change is rarely seen in chronic or subclinical infection where continued monitoring is advised. Many early infections undergo self cure after a few days and do not necessitate treatment (which removes the cow from the milking herd until antibiotic residues are cleared from the milk resulting in a loss of hundreds of Kgs of milk). As a result sudden very high increases of either TLC or N count would prompt SR4 kinetics or immediate treatment while moderate changes in TLC and N are best monitored to see if they result in self cures (recovery).

Once the TLC or N exceeds 1,000 kinetics or immediate treatment is advised depending on exactly what the count is and whether or not the animal is showing systemic signs of disease i.e., fever, loss of appetite, loss of rumen contractions. Presence of systemic signs call for immediate treatment or culling of the animal from the herd. When possible it is best to capture several 24 hour MDLC in order to establish the period of moderate increasing cell count (subclinical period), the period of rapidly increasing cell count, the peak cell count, the period of peak plateau, and the final baseline count. The presence or absence of a subclinical period before a period of rapidly increasing cell count is indicative of gram positive infections; absence of this subclinical period is indicative of gram negative infections (all of which are generally treated the same). The peak height of the rapid increasing cell count is indicative of the type of gram negative or gram positive infection. The length between the beginning of the subclinical period and the period of rapid increasing cell count is indicative of different gram positive infections as is the length of the peak plateau and the final post recovery baseline.

Another important factor used to diagnose species-specific mastitis is the timing of entry into the milk of the various leukocyte populations. E.coli infection is heralded by the rapid recruitment of N into milk in 2 to 4 hours, followed by the recruitment of L and M within 6 hours of infection. This rapid recruitment of all three leukocyte populations is also typical of other gram negative bacteria (which are all treated with the same antibiotics). Gram positive bacteria have more species-specific changes in leukocyte recruitment with delays seen in the recruitment of macrophages and lymphocytes, sometimes by days after infection.

Thus SR3 is used while TLC is changing between 150 and 800. During this time the count may go back down to baseline or it may increase above 800 in which case you have at least 24 hours of kinetics in SR4 in which to base a species-specific diagnosis.

If at any time the TLC or N count begins to decrease they should be monitored using SR2 for recovery. This subroutine will indicate when it is indicated to establish a new baseline.

At any time usually >80% of all quarters of cows in a herd have leukocyte values at baseline. The use of SR2-4 should be an anomaly.

### Key to Figures:

TLC - Total Leukocyte Count
NC - Neutrophil Count
MC - Macrophage Count
LC - Lymphocyte Count
%N - Percent Neutrophils
%M - Percent Macrophages
%L - Percent Lymphocytes
↑ - Increasing
↓ - Decreasing
- - No Change in Number or Percentage
Rx - Treatment
Cull - Remove Animal from Herd
SC - Subclinical Infection
Gm+ - Bacteria is Gram Stain Positive
Gm- - Bacteria is Gram Stain Positive

## Claims

1. A computer-implemented method for diagnosing mastitis in a lactating and preferably bovine animal, the method comprising:
- recording data, the data having been obtained from analyzing a series of milk samples, the series of milk samples in turn having been obtained by periodically milking an animal, the data including total leukocyte count (TLC) or somatic cell count (SCC) as well as differential leukocyte counts and changes in these counts between subsequent samples, wherein the differential leukocyte counts include the lymphocyte count (L), the macrophage count (M) and the neutrophil count (N); and
- evaluating the recorded data to obtain information on the presence of mastitis, on the microorganism responsible for mastitis and, optionally, on the stage of mastitis;
wherein evaluating the recorded data includes:
determining mean values and confidence intervals for the TLC or SCC and each of the differential leukocyte counts L, M and N;
assigning the samples to threshold groups based on the mean value of the TLC or the SCC;
evaluating whether the TLC or SCC of a new sample enters a higher threshold group and, if applicable, which threshold group has been entered; and
evaluating whether a threshold group has been exceeded and, if so, evaluating the recorded data to obtain information on the microorganism responsible for mastitis, wherein the prediction of a specific microorganism is made based on information regarding peak TLC or SCC in previous samples, length of the plateau before the TLC or SCC decreases, and the change in percentages of N, M and L during a number of previous samplings.

2. The method of claim 1, wherein evaluating the recorded data further includes evaluating whether the counts of a new sample remain within the respective confidence intervals and, if applicable, identifying a deviation.

3. The method of claim 2, wherein evaluating the recorded data further includes evaluating the deviation to identify acute infection, chronic infection or recovery.

4. The method of claim 3, wherein the method further includes identifying and outputting recommendations on whether to dump the milk or treat the animal on the basis of the result of the evaluation of the deviation.

5. The method of any preceding claim, wherein evaluating the recorded data further includes evaluating whether the TLC or SCC of a new sample enters a higher threshold group and, if applicable, which threshold group has been entered.

6. The method of claim 5, wherein evaluating the recorded data further includes evaluating the change of threshold groups to identify acute infection, chronic infection or recovery.

7. The method of claim 6, wherein the method further includes identifying and outputting recommendations on whether to dump the milk or treat the animal on the basis of the result of the evaluating the change of threshold groups.

8. The method of any preceding claim, wherein the period for milking is once or twice daily.

9. The method of any preceding claim, wherein the samples constitute composite samples comprising milk from different mammary gland quarters.

10. The method of any one of claims 1-8, wherein individual series of samples have been obtained for each of the four quarters of a cow.

11. A data storage medium comprising a computer-readable program configured to carry out the method of any preceding claim.

12. A computer configured to carry out the method of any one of claims 1-10.

## Patentansprüche

1. Computer-implementiertes Verfahren zur Diagnose von Mastitis bei einem laktierenden Tier, bevorzugt einem Hornträger, wobei das Verfahren umfasst:
- Aufzeichnen von Daten, wobei die Daten erhalten wurden aus der Analyse einer Reihe von Milchproben, die wiederum erhalten wurden durch periodisches Melken eines Tieres, wobei die Daten eine Gesamtleukozytenzahl (TLC) oder eine Anzahl somatischer Zellen (SCC) sowie unterschiedliche Leukozytenzahlen und Veränderungen in diesen Zahlen zwischen aufeinanderfolgenden Proben umfassen, wobei die unterschiedlichen Leukozytenzahlen die Lymphozythenzahl (L), die Makrophagenzahl (M) und die Neutrophilenzahl (N) umfassen; und
- Auswerten der aufgezeichneten Daten, um Informationen über das Vorhandensein von Mastitis, über die für die Mastitis verantwortlichen Mikroorganismen, und optional, über das Stadium der Mastitis zu erhalten;
wobei das Auswerten der aufgezeichneten Daten umfasst:
Ermitteln von Mittelwerten und Konfidenzintervallen für die TLC oder die SCC und jede der unterschiedlichen Leukozytenzahlen L, M, und N;
Zuordnen der Proben zu Schwellenwertgruppen basierend auf dem Mittelwert der TLC oder der SCC;
Auswerten, ob entweder die TLC oder die SCC einer neuen Probe in eine höhere Schwellenwertgruppe eintritt, und, gegebenenfalls, welche Schwellenwertgruppe betreten wurde; und
Auswerten, ob eine Schwellenwertgruppe überschritten wurde, und, wenn dies der Fall ist, Auswerten der aufgezeichneten Daten, um Informationen über die für die Mastitis verantwortlichen Mikroorganismen zu erhalten, wobei das Prognostizieren eines konkreten Mikroorganismus auf Grundlage von Informationen hinsichtlich einer Peak-TLC oder -SCC in früheren Proben, der Länge des Plateau vor der Abnahme der TLC oder der SCC, und der Veränderung der Prozentsätze von N, M, und L während einer Anzahl früherer Probenentnahmen erfolgt.

2. Verfahren nach Anspruch 1, wobei das Auswerten der aufgezeichneten Daten ferner das Auswerten umfasst, ob die Zahlen einer neuen Probe innerhalb der jeweiligen Konfidenzintervalle bleiben, und, falls zutreffend, Ermitteln einer Abweichung.

3. Verfahren nach Anspruch 2, wobei das Auswerten der aufgezeichneten Daten ferner das Auswerten der Abweichung zum Ermitteln einer akuten Infektion, einer chronischen Infektion, oder einer Genesung umfasst.

4. Verfahren nach Anspruch 3, wobei das Verfahren auf Grundlage des Ergebnisses der Auswertung der Abweichung ferner das Ermitteln und Ausgeben von Empfehlungen umfasst, ob die Milch entsorgt oder das Tier behandelt werden soll.

5. Verfahren nach einem der vorstehenden Ansprüche, wobei das Auswerten der aufgezeichneten Daten ferner das Auswerten umfasst, ob die TLC oder SCC einer neuen Probe in eine höhere Schwellenwertgruppe eintritt, und, gegebenenfalls, in welche Schwellenwertgruppe sie eingetreten ist.

6. Verfahren nach Anspruch 5, wobei das Auswerten der aufgezeichneten Daten ferner das Auswerten der Änderung von Schwellenwertgruppen zum Erkennen einer akuten Infektion, einer chronischen Infektion oder einer Genesung umfasst.

7. Verfahren nach Anspruch 6, wobei das Verfahren auf Grundlage des Ergebnisses der Auswertung der Änderung der Schwellenwertgruppen ferner das Erkennen und Ausgeben von Empfehlungen umfasst, ob die Milch entsorgt oder das Tier behandelt werden soll.

8. Verfahren nach einem der vorstehenden Ansprüche, wobei der Zeitraum zum Melken einmal oder zweimal täglich ist.

9. Verfahren nach einem der vorstehenden Ansprüche, wobei die Proben zusammengesetzte Proben darstellen, die Milch aus unterschiedlichen Drüsenvierteln eines Säugetiers beinhalten.

10. Verfahren nach einem der Ansprüche 1 bis 8, wobei einzelne Probenreihen für jedes der vier Viertel einer Kuh erhalten wurden.

11. Datenspeichermedium, aufweisend ein computerlesbares Programm, das zum Durchführen des Verfahrens nach einem der vorstehenden Ansprüche eingerichtet ist.

12. Computer, eingerichtet zum Durchführen des Verfahrens nach einem der Ansprüche 1 bis 10.

## Revendications

1. Procédé mis en œuvre par ordinateur pour le diagnostic d'une mammite chez un animal en lactation et de préférence un animal bovin, le procédé comprenant :
- l'enregistrement des données, les données ayant été obtenues à partir de l'analyse d'une série d'échantillons de lait, la série d'échantillons de lait ayant à son tour été obtenue par traite périodique d'un animal, les données incluant la numération globale des leucocytes (TLC) ou la numération des cellules somatiques (SCC) ainsi que les numérations différentiels des leucocytes et les changements de ces numérations entre des échantillons subséquents, dans lequel les numérations différentiels des leucocytes incluent la numération des lymphocytes (L), la numération des macrophages (M) et la numération des neutrophiles (N) ; et
- l'évaluation des données enregistrées pour obtenir des informations sur la présence de mammite, sur le microorganisme responsable de la mammite et, facultativement, sur le stade de la mammite ;
dans lequel l'évaluation des données enregistrées comprend:
la détermination des valeurs moyennes et des intervalles de confiance pour le TLC ou le SCC et chacun des numérations différentielles des leucocytes L, M et N ;
l'attribution des échantillons à des groupes de seuil sur la base de la valeur moyenne du TLC ou du SCC ;
l'évaluation si le TLC ou le SCC d'un nouvel échantillon entre dans un groupe de seuil plus élevé et, le cas échéant, dans quel groupe de seuil il est entré ; et
l'évaluation si un groupe de seuil a été dépassé et, le cas échéant, le fait l'évaluation des données enregistrées pour obtenir des informations sur le microorganisme responsable de la mammite, où la prédiction d'un microorganisme spécifique est faite sur la base des informations concernant le TLC ou le SCC de pic dans des échantillons anciens, la longueur du plateau avant que le TLC ou le SCC ne diminue, et le changement des pourcentages de N, M et L pendant un nombre d'échantillons anciens.

2. Procédé selon la revendication 1, dans lequel l'évaluation des données enregistrées inclut en outre le fait d'évaluer si les numérations d'un nouvel échantillon restent au sein des intervalles de confiance respectifs et, le cas échéant, l'identification d'une déviation.

3. Procédé selon la revendication 2, dans lequel l'évaluation des données enregistrées inclut en outre l'évaluation de la déviation pour identifier une infection aiguë, une infection chronique ou un rétablissement.

4. Procédé selon la revendication 3, dans lequel le procédé inclut en outre l'identification et l'émission des recommandations indiquant si le lait est à jeter ou si l'animal est à traiter sur la base du résultat de l'évaluation de la déviation.

5. Procédé selon une quelconque des revendication précédentes, dans lequel l'évaluation des données enregistrées inclut en outre l'évaluation si le TLC ou le SCC d'un nouvel échantillon entre dans un groupe de seuil plus élevé et, le cas échéant, dans quel groupe seuil il est entré.

6. Procédé selon la revendication 5, dans lequel l'évaluation des données enregistrées inclut en outre l'évaluation le changement des groupes de seuil pour identifier une infection aiguë, une infection chronique ou un rétablissement.

7. Procédé selon la revendication 6, où le procédé inclut en outre l'identification et l'émission des recommandations indiquant si le lait est à jeter ou si l'animal est à traiter sur la base du résultat de l'évaluation de la déviation de groupes de seuil.

8. Procédé selon une quelconque des revendications précédentes, dans lequel la période de traite est une ou deux fois par jour.

9. Procédé selon une quelconque des revendications précédentes, dans lequel les échantillons constituent des échantillons composites comprenant du lait provenant de différents quartiers de glande mammaire.

10. Procédé selon l'une quelconque des revendications 1 à 8, dans lequel des séries individuelles d'échantillons ont été obtenues pour chacun des quatre quartiers d'une vache.

11. Support de stockage de données comprenant un programme lisible par ordinateur configuré pour effectuer le procédé selon l'une quelconque des revendications précédentes.

12. Ordinateur configuré pour effectuer le procédé selon l'une quelconque des revendications 1 à 10.
